# EUROPEAN PATENT SPECIFICATION

(11) **EP 4 231 975 B1**
(45) Date of publication and mention of the grant of the patent: **05.11.2025**
(21) Application number: 21881368.1
(22) Date of filing: 12.10.2021
(51) Int. Cl.: A61F 5/41, A61N 1/36, A61H 19/00, A61N 5/06, A61H 23/02

(54) **VASODILATION AND NEOVASCULARIZATION METHODS AND APPARATUS FOR ERECTILE DYSFUNCTION**
VERFAHREN ZUR VASODILATATION UND NEOVASKULARISATION UND GERÄT ZUR EREKTILEN DYSFUNKTION
PROCÉDÉS ET APPAREIL DE VASO-DILATATION ET DE NÉOVASCULARISATION POUR DYSFONCTIONNEMENT ÉRECTILE

(30) Priority: 22.10.2020 AU 2020903831
(43) Date of publication of application: 30.08.2023
(73) Proprietor: Cosmetic Edge Pty Ltd, Kew, Victoria 3101 (AU)
(72) Inventor: TAHGHIGHI JAFARZADEH, Masoud, Kew, Victoria 3101 (AU)
(74) Representative: K&L Gates LLP
(86) International application number: PCT/AU2021/051191
(87) International publication number: WO 2022/082255

(56) References cited:
- WO-A1-2004/045411
- WO-A1-2020/041830
- WO-A2-2017/089887
- CN-A- 111 407 620
- GB-A- 2 540 746
- KR-A- 20060 127 450
- KR-A- 20200 111 605
- KR-B1- 101 782 357
- KR-U- 20170 001 199
- US-A1- 2014 303 693
- US-A1- 2015 141 881

## Description

### TECHNICAL FIELD

The invention relates to an erectile dysfunction treatment apparatus. The invention further relates to a method of treating erectile dysfunction.

### BACKGROUND

Erectile dysfunction, otherwise known as male impotence, refers to an inability of an individual to obtain and maintain a penile erection that is firm enough for sexual intercourse. Temporary or infrequent erection trouble is not necessarily a cause for concern. However, ongoing or persistent erectile dysfunction can be problematic for males. The inability to maintain a penile erection can lead to stress, self-confidence issues, and relationship problems. Further, erectile dysfunction can be a sign of underlying health issues and/or a risk factor for heart disease.

Recent studies have demonstrated that vasoconstriction in the erectile vasculature of a penis is a significant cause of erectile dysfunction. Vasoconstriction causes limited access of blood to the penis, which makes it virtually impossible to maintain an erection. A need accordingly exists for a device or treatment that provides vasodilation to improve blood flow through penile blood vessels. Document US2014/303693 A discloses a wearable device for photo-therapy or light therapy applications to male genitalia.

The present invention was conceived with these shortcomings in mind.

### SUMMARY

The problems described in the prior art have been solved with an apparatus according to claim 1. The example methods and apparatus disclosed herein are directed to a non-invasive male therapeutic device for treating erectile dysfunction. The example methods and apparatus are configured to provide vasodilation and neovascularization within a penile vessels and tissues. The vasodilation portion of the treatment increases penile blood vessel diameter or effective blood flow diameter within penile blood vessels to improve blood flow. Further, the neovascularization portion of the treatment causes new blood vessels to form in penile tissue. Together, the vasodilation and neovascularization treatments provided by the disclosed methods and apparatus improve blood flow through a patient's penis, thereby enabling a stronger, longer lasting erection for sexual intercourse.

The example methods and apparatus comprise a seat-like device that is configured to cradle at least a portion of a patient's perineal and penile regions. The seat-like device includes one or more acoustic wave actuators that cause microscopic trauma within the targeted tissue. The microscopic trauma causes new blood vessels to form via neovascularization. The seat-like device also includes one or more vibration actuators and one or more LEDs configured to project low levels of light. Together, the vibration and light are directed to fine blood vessels in the perineal and penile regions, causing vasodilation.

The example methods and apparatus are configured to provide vasodilation and neovascularization treatments at least two to three times a week for a total of fifteen to thirty sessions. Each session may be between five minutes and twenty minutes. After initial treatment, a patient may use the methods and apparatus disclosed herein once a week to maintain the therapeutic results.

In a first aspect, the invention provides an erectile dysfunction treatment apparatus comprising: a pad adapted to contact a hind region of a patient, the pad including an acoustic wave actuator; a pouch connected to the pad, the pouch configured to wrap around a penile shaft of the patient, the pouch including at least one primary vibrating pad, and at least one primary LED; and a control unit electrically coupled to the acoustic wave actuator, the at least one vibrating pad, and the at least one LED, the control unit configured to provide first actuation signals to the acoustic wave actuator for performing a neovascularization treatment, and configured to provide second actuation signals to the at least one primary vibrating pad, and the at least one primary LED for performing a vasodilation treatment. The pad may comprise a perineal section and a penile section. The penile section of the pad comprises a first recess section and a second recess section together forming a penile shape and configured to respectively cradle a scrotum of the patient and at least a portion of the penile shaft of the patient.

In a second aspect, the invention provides an erectile dysfunction treatment apparatus comprising: a pad adapted to contact a hind region of a patient, the pad including perineal and penile sections; an acoustic wave actuator included in the pad at the peroneal section; first and second recess sections located at the penile section, the first and second recess sections configured to respectively cradle a scrotum of the patient and at least a portion of a penile shaft of the patient; and a pouch connected to the pad at the second recess section, the pouch configured to wrap around the penile shaft, the pouch including at least one primary vibrating pad, and at least one primary LED.

It is also disclosed a method of treating erectile dysfunction with a treatment apparatus comprising: a pad adapted to contact a hind region of a patient; an acoustic wave actuator included in the pad; a pouch connected to the pad, the pouch configured to wrap around a penile shaft of the patient, the pouch including at least one primary vibrating pad, and at least one primary LED; and a control unit electrically coupled to the acoustic wave actuator, the at least one vibrating pad, and the at least one LED, wherein the control unit is configured to provide first actuation signals to the acoustic wave actuator for performing a neovascularization treatment, and configured to provide second actuation signals to the at least one primary vibrating pad and the at least one primary LED for performing a vasodilation treatment.

Additional features and advantages are described in, and will be apparent from, the following Detailed Description and the Figures. The features and advantages described herein are not all-inclusive and, in particular, many additional features and advantages will be apparent to one of ordinary skill in the art in view of the figures and description. Also, any particular embodiment does not have to have all of the advantages listed herein and it is expressly contemplated to claim individual advantageous embodiments separately. Moreover, it should be noted that the language used in the specification has been selected principally for readability and instructional purposes, and not to limit the scope of the inventive subject matter.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, a limited number of the exemplary methods and materials are described herein.

### BRIEF DESCRIPTION OF THE FIGURES

Exemplary embodiments of the invention are described below by way of example only, and not by way of limitation. Referring now to the accompanying drawings in which like numerals indicate like elements throughout the several figures:
Fig. 1 shows a diagram of an erectile dysfunction treatment environment including a treatment pad operating with a user device, according to an example embodiment of the present disclosure.
Fig. 2A shows a diagram of the treatment pad of Fig. 1, according to an example embodiment of the present disclosure.
Fig. 2B shows the treatment pad of Fig. 2A, with a dashed outline of a user in situ on the treatment pad.
Fig. 3 is a lateral cross-section of a flaccid penis indicating vasodilation and neovascularization treatments provided by the treatment pad of Figs. 1 and 2, according to an example embodiment of the present disclosure.
Fig. 4A is a transverse cross-sectional view of a flaccid penis prior to vasodilation and neovascularization by the treatment pad of Figs. 1 and 2.
Fig. 4B is a lateral cross-sectional view of the flaccid penis of Fig 4A.
Fig. 4C is a lateral cross-sectional view of an erect penis after vasodilation and neovascularization by the treatment pad of Figs. 1 and 2.
Fig. 4D is a transverse cross-sectional view of the erect penis of Fig. 4C.

Embodiments will now be described more fully hereinafter with reference to the accompanying drawings, in which various embodiments, although not the only possible embodiments, of the invention are shown. The invention defined in the appended claims may be embodied in many different forms and should not be construed as being limited to the embodiments described below.

### DETAILED DESCRIPTION

The present disclosure relates in general to a method and apparatus for providing therapeutic neovascularization and vasodilation treatments for erectile dysfunction. The disclosed method and apparatus are configured to provide acoustic vibration for inducing neovascularization within a patient's perineal body (e.g., a central tendon of the perineum). The perineal body helps strengthen a patient's pelvic floor. A strengthened pelvic floor helps create and maintain a penile erection.

The disclosed method and apparatus are configured to provide oscillating vibration actuators and/or light emission (low level light therapy) via one or more LEDs for inducing vasodilation within at least a portion of a penile shaft. Opening or expanding penile blood vessels improves blood flow through the tissue, thereby helping to create and maintain a penile erection.

Reference is made herein to the methods and devices providing vasodilation and neovascularization treatments. It should be appreciated that in some embodiments, the methods and apparatus disclosed herein may only provide either neovascularization treatment or vasodilation treatment. In these other embodiments, the features described for the other, not administered treatment may not be present on the device or may not be used during a therapy session.

Throughout the disclosure, reference is made to user devices. As disclosed herein, a user device can include any cellphone, smartphone, personal digital assistant ("PDA"), mobile device, tablet computer, computer, laptop, server, processor, console, gaming system, multimedia receiver, workstation, disk storage device, or any other computing device.

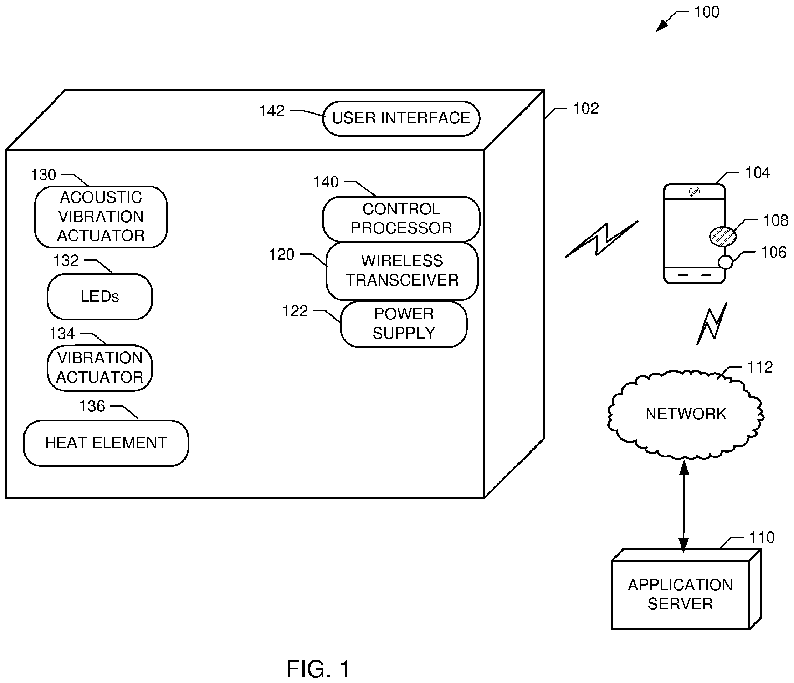

Fig. 1 is a diagram of an example erectile dysfunction treatment environment 100 including a treatment pad 102 and a user device 104. The example user device 104 includes a processor 106 that is configured to execute an erectile dysfunction application 108. The application is defined or specified by one or more instructions stored in a memory of the user device 104. Execution of the instructions by the processor 106 cause the processor 106 to perform the operations described herein in connection with the application 108.

The example user device 104 is configured to communicatively couple to the treatment pad 102 via a wired or wireless connection. A wired connection may include a USB connection, a Lightning^{®} connection, etc. A wireless connection may include a Bluetooth^{®} connection, a Zigbee^{®} connection, an NFC connection, a Wi-Fi connection, etc.

In some embodiments, the user device 104 may be omitted. In these embodiments, the operation of the treatment pad 102 may be self-contained.

In some embodiments, the application 108 may be communicatively coupled to an application server 110 via a network 112 (e.g., any wide area network, cellular network, or combinations thereof). The application server 110 may be configured to store template recommendations, therapies, and/or treatments for access by the application 108. Such recommendations may be newly created as additional uses/treatments of the device 102 are determined/approved. Further, the recommendations may be modified overtime based on feedback from a population of users. The application 108 is configured to download the recommendations for customization to a user's condition and/or past treatment history.

The example treatment pad 102 includes a wireless (or wired) transceiver 120 for communication with the user device 104. In some embodiments, the transceiver 120 may provide for pairing with the user device 104. The example treatment pad 102 also includes a power supply 122. The example power supply 122 may include one or more batteries and/or a connection to a power outlet. The power supply 122 provides power for the treatment pad 102.

The example treatment pad 102 includes at least one acoustic vibration actuator 130. The actuator 130 can include a device that causes a steel ball to vibrate or otherwise bounce within a housing to produce acoustic waves. The acoustic waves are configured to target a perineal region of a patient for promoting neovascularization.

In some embodiments the acoustic vibration actuator 130 can be substituted or complemented by an electro-muscle stimulation device (EMS). EMS can also be referred to as neuromuscular electrical stimulation (NMES). As the name suggests electrical impulses are used to mimic the action of signals coming from neurons (cells in the nervous system) to stimulate muscle contraction to activate and train weakened muscles. The electrical impulses can also be used to target nerves. The electrical impulses are delivered by an electrode 131 to the desired treatment area. The electrode 131 can be co-located with the actuator 130 or located centrally or peripherally thereto, to target the perineal region of the patient. The EMS can be configured to induce repetitive, fast contraction bursts, followed by muscle relaxation, leading to strengthening of perineal and rectal muscle network, involved in erectile dysfunction, stress urinary and faecal incontinence. The EMS electrode 131 feature can assist in the muscle recovery, where neuron signals get sent to targeted muscles to make them contract. By causing repeated muscle contractions, blood flow improves, helping repair injured muscles. The EMS electrode 131 can be formed as a stimulation ring surrounding the vibration actuator 130.

The example treatment pad 102 also includes at least one light or other LED 132 and at least one vibration actuator 134. The at least one LED 132 may provide low level light that promotes vasodilation. At least some of the lights or LEDs 132 may emit light at a 685 nm wavelength while other of the lights or LEDs 132 may emit light at a 780 nm wavelength. It should be appreciated that the LEDs 132 may emit light at additional or fewer wavelengths. In some embodiments, the treatment pad 102 may include 10, 20, 30, 40, etc. lights or LEDs.

The treatment pad 102 may further include one or more vibration actuators 134. The example vibration actuators 134 include vibrating oscillators, which produce vibrations that travel through patient tissue. The actuators 134 may vibrate at the same frequency or at different frequencies. The actuator 134 may operate at 7,000 RPM to generate waves for inducing penile tissue vibration.

In some embodiments, the treatment pad 102 may include a heat element 136. The example heat element 136 may include one or more heating coils or other structure that generates heat upon from a received electrical current. The heat element 136 may warm the treatment pad 102, providing comfort to a patient. The heat element 136 may also enhance vasodilation and/or neovascularization. The heat element 136 may be configured to heat to a temperature between 30 degrees centigrade to 50 degrees centigrade. In other embodiments, the heat element 136 includes a radio transmitter configured to provide localized tissue heating via radiofrequencies. The element 136 may provide heating using a frequency between 100 kHz-300 GHz.

The example components 120 to 136 may be controlled by a control processor 140. The example control processor 140 is configured to send activation signals to each of the components 120 to 136 when activation is needed. For example, for a treatment, the control processor 140 may cause the acoustic vibration actuator 130 to generate acoustic waves at one or more frequencies. Additionally or alternatively, the control processor 140 may cause the LEDs 132 to emit light fur certain time durations and/or cause the vibration actuator(s) 134 to vibrate at one or more specified frequencies. The example control processor 140 is also configured to transmit signals for activation of the heat element 136.

In some embodiments, the control processor 140 is configured to operate according to one or more treatment profiles. The profiles may be stored to a memory device (not shown). The treatment profiles may be stored at a time of manufacture, and selected by a patient using a user interface 142. Additionally or alternatively, treatment profiles may be manually set by the patient or a clinician using the user interface 142. Moreover, in some instances, treatment profiles may be received from the application 108 on the user device 104.

Each treatment profile may specify which of the components 130 o 136 are to be actuated, and a duration of their actuation for a session. The treatment profile may also specify a vibration intensity or amplitude for the actuators 130 and 134. The treatment profile may further specify a light intensity for the LEDs 132 and a temperature for the heat element 136. The profile may also specify changes in light brightness and/or vibration intensity. Further, the profile may specify different waveform shapes to produce different types of vibrations.

In some instances, the control processor 140 may track a patient's complete treatment. For instance, during a first portion of a treatment, the control processor 140 may select a profile having a duration between ten and twenty minutes, which has a certain vibration and light intensity pattern. The control processor 140 selects this profile for the first fifteen to thirty sessions, if those sessions are performed two to three times a week. Thereafter, the control processor 140 selects a second profile with a different duration, vibration intensity, and/or light intensity.

In some embodiments, the processor 140 may transmit indications of which profile was administered to the application 108 on the user device 104. This may provide for tracking therapy progress by the patient and/or the clinician. The application 108 may recommend or transmit modified profiles based on a patient's usage frequency and/or feedback regarding progress on their erectile dysfunction.

The example user interface 140 may include one or more buttons or switches, such as a power button, a communication pairing button, a light button for manual activation of the LEDs 132, and/or a start/pause/stop control for beginning, pausing, and stopping a treatment or therapy. In some embodiments, the user interface 140 may be integrated with the application 140 on the user device 104.

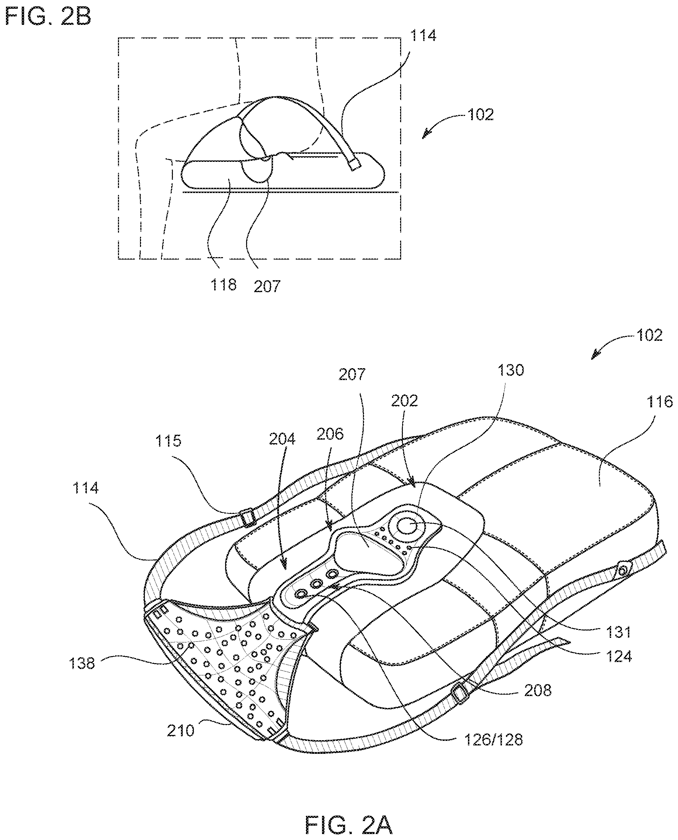

Fig. 2A shows a diagram of the treatment pad 102 of Fig. 1, according to an example embodiment of the present disclosure. The treatment pad 102 is contoured to form a seat-shape or saddle-shape and is configured to contact a hind region of a patient (as illustrated in Fig. 2B). The treatment pad 102 includes a memory foam core 118 with a polyester cover 116. The cover 116 can be made from a variety of fabrics and can be configured to be removable for cleaning or replacement. The treatment pad 102 may have a length of 24 inches, a width of 18 inches, and a height of 5 inches. As shown in Fig. 2, the treatment pad includes a perineal section 202 and a penile section 204.

The perineal section 202 is configured to contact a perineal region of a patient. The example perineal section 202 includes at least one acoustic wave actuator 130. Further, in some embodiments, the perineal section 202 can include one or more vibrating pads 134 and/or one or more LEDs 132 within an LED/vibrator array 138.

The penile section 204 is configured to contact a penile region 300 of a patient, including a scrotum and penile shaft. The penile section 204 includes a first recess section 206 comprising a padded protective shell 207 to cradle the scrotum. The first recess section 206 is arranged in a triangular shape and allows the scrotum to be cradled therein. The first recess section 206 can include one or more LEDs 124 arranged in proximity to the acoustic wave actuator 130. The LEDs 124 can emit light at a wavelength between 685 nm and 780 nm.

The penile section 204 also includes a second recess section 208, which is shallower than the first recess section 206. The second recess section 208 is elongate and concaved extending into the pad 102 and extending from the first recessed section 206 towards an edge of the pad 102. The second recess section 208 is configured to cradle at least a portion of a patient's penile shaft. In some embodiments, the second recess section 208 can include one or more vibrating pads 128 and/or one or more LEDs 128. Together, the first and second recess sections 206 and 208 have a penile shape. The LEDs 128 can emit light at a wavelength between 685 nm and 780 nm.

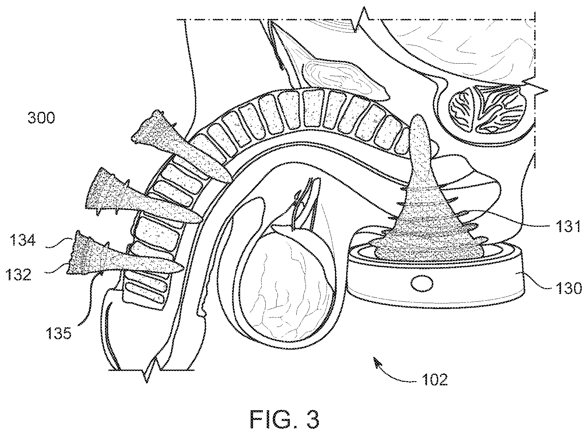

The treatment pad 102 also includes a pouch 210, which is connected to the pad 102 at an end of the second recess section 208. The pouch 210 is flexible and can be made from neoprene and is configured to be stretched or otherwise pulled around at least a portion of the penile shaft. The pouch 210 includes an LED/vibrator array 138 comprising one or more vibrating pads 134 and/or one or more LEDs 132 (shown in Figure 3).

In the illustrated example of Fig. 2A, the pouch 210 includes 40 LEDs. In some embodiments, the pouch 210 is configured to rotate at a connection point with the pad 102, thereby rotating back toward the patient to cover the entirety of the penile shaft. The pouch 210 can be connected to straps 114 to hold the pouch 210 in place around the penile shaft during treatments. The straps 114 can be resilient and can be formed from an elastic material. Additionally or alternatively, the straps 114 can include an adjustor or buckle 115.

### Treatment Embodiments

Fig. 3 is a lateral cross-section of a flaccid penis during a vasodilation and neovascularization treatment provided by the treatment pad 102 of Figs. 1 and 2, according to an example embodiment of the present disclosure. In the illustrated example, the acoustic vibration actuator 130 provides acoustic wave energy (diagrammatically illustrated as a wave 131 penetrating a perineal region) to the perineal region of a patient. The acoustic wave energy 131 causes microscopic tissue trauma. To combat the induced trauma, a patient's body causes new blood vessels to grow in the affected tissue, thereby improving blood flow.

Further, LEDs 132 and vibration oscillators 134 of the array 138 surround the penile shaft and provide mechanical oscillating vibration energy and light in the 685 and 780 nm wavelength range. The pouch 210 has been removed from Fig. 3 for clarity, however, it is the flexible pouch 210 that supports the LEDs 132 and vibration oscillators 134 in proximity to the penile shaft. Waves 135 of light and/or vibration are schematically illustrated penetrating the penile shaft during a vasodilation and neovascularization treatment. Together, the components 130 to 135 provide for vasodilation and neovascularization of blood vessels in the perineal and penile region 300 of the patient. The light and/or oscillating vibrations can prevent the breakdown of nitric oxide in a patient's penile blood vessels. Nitric oxide is a chemical messenger that promotes the relaxation and opening of blood vessels that bleed to erect tissue in a penis. Under the influence of nitric oxide, these vessels expand and stay dilated. Increased blood flow makes erectile tissue swell, and compress the veins that carry blood out of the penis, resulting in a full erection.

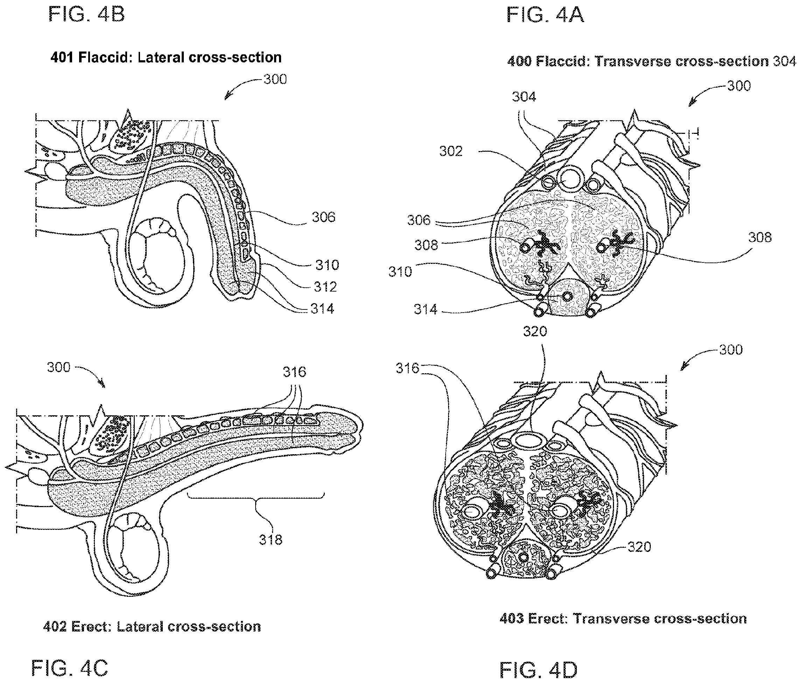

Figs. 4A-4B shows cross-sections of the penile region 300 before treatment using the treatment pad 102 of Figs. 1 and 2, according to an example embodiment of the present disclosure. Fig. 4A illustrates a transverse cross-section of a flaccid penis (400), indicating a patient experiencing erectile dysfunction and having uncompressed penile veins 304, a spongy urethra 310, and less blood flow through the corpora cavernosa 306. In Fig. 4A the deep dorsal vein 302 is illustrated in an uncompressed form having a generally circular cross-section. Additionally the cavernosal arteries 308 are illustrated with circular cross-section in the flaccid penis.

The corpus spongiosum 314 is a column of spongy tissue that runs through the penile shaft and glans. The foreskin or prepuce 312 covers the glans. The corpus spongiosum 314 is illustrated in Fig. 4B surrounding the urethra 310. The blood vessels within the corpus spongiosum 314 need to fill with blood to produce an erection and keep the urethra open during the erection.

Figs. 4B-4C show the penile region 300 and tissue after at least ten sessions using the treatment pad 102 of Figs. 1 and 2. As shown, the vasodilation helps the cavernosal arteries to dilate 316, which engorges corporal tissue with blood 318. This engorging causes the corporal tissue to swell 318, which causes the penis to become erect. The engorged corporal tissue 318 compresses penile veins and venules 320, thereby maintaining the erection. Further, neovascularization in the perineal region helps form new blood vessels, which increases strength of the fibro-muscular perineum and strengths the pelvic floor for maintaining an erection.

### Conclusion

It will be appreciated that all of the disclosed methods and procedures described herein can be implemented using one or more computer programs or components. These components may be provided as a series of computer instructions on any conventional computer-readable medium, including RAM, ROM, flash memory, magnetic or optical disks, optical memory, or other storage media. The instructions may be configured to be executed by a processor, which when executing the series of computer instructions performs or facilitates the performance of all or part of the disclosed methods and procedures.

It should be understood that various changes and modifications to the example embodiments described herein will be apparent to those skilled in the art. Such changes and modifications can be made without departing from the scope of the present subject matter and without diminishing its intended advantages. It is therefore intended that such changes and modifications be covered by the appended claims.

As used herein, "treat", "treating" or "treatment" of a disorder or condition (such as erectile dysfunction, and related conditions) means accomplishing one or more of the following: (a) reducing the severity and/or duration of the disorder/condition; (b) limiting or preventing development of symptoms characteristic of the disorder(s)/condition(s); (c) inhibiting worsening of symptoms characteristic of the disorder(s)/condition(s) being treated; (d) limiting or preventing recurrence of the disorder(s)/condition(s) in patients that have previously had the disorder(s)/condition(s); and (e) limiting or preventing recurrence of symptoms in patients that were previously symptomatic for the disorder(s)/condition(s). As used herein, "prevent", "preventing", "prevention", or "prophylaxis" of a disorder or condition means preventing the disorder/condition occurring in subject.

It is to be understood that, if any prior art publication is referred to herein, such reference does not constitute an admission that the publication forms a part of the common general knowledge in the art, in Australia or any other country.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

### LEGEND

| **Ref.** # | **Description** | **Ref.** # | **Description** |
|---|---|---|---|
| 100 | Treatment environment | 136 | Heat element |
| 102 | Pad | 138 | LED/vibrator array |
| 104 | Device | 140 | Control processor |
| 106 | Processor | 142 | User interface |
| 108 | Application | | |
| 110 | Server | 202 | Perineal section |
| 112 | Network | 204 | Penile section |
| 114 | Elastic strap | 206 | First recess section |
| 115 | Adjustor | 208 | Second recess section |
| 116 | Polyester cover | 210 | Pouch |
| 118 | Memory foam core | | |
| | | 300 | Penile region |
| 120 | Transceiver | 302 | Deep dorsal vein |
| 122 | Power supply | 304 | Penile venules (uncompressed) |
| 124 | First recess LEDs | 306 | Corpora cavernosa |
| 126 | Second recess LEDs | 308 | Cavernosal arteries |
| 128 | Second recess vibration pads | 310 | Spongy urethra |
| 130 | Acoustic wave actuator | 312 | Prepuce |
| 131 | Acoustic wave | 314 | Corpus spongiosum |
| 132 | LED | 316 | Dilated cavernosal arteries |
| 134 | Vibration actuator | 318 | Engorged/swollen corporal tissue |
| 135 | Vibrational wave | 320 | Compressed penile veins |
| 400 | Transverse penile cross-section: Flaccid | 402 | Lateral penile cross-section: Erect |
| 401 | Lateral penile cross-section: Flaccid | 403 | Transverse penile cross-section: Erect |

## Claims

1. An erectile dysfunction treatment apparatus comprising:
a pad adapted to contact a hind region of a patient, the pad comprising a perineal section and a penile section is contoured to form a seat-shape or saddle-shape, the perineal section including an acoustic wave actuator;
a pouch connected to the penile section of the pad, the pouch configured to wrap around a penile shaft of the patient, the pouch including at least one primary vibrating pad, and at least one primary LED;
a control unit electrically coupled to the acoustic wave actuator, the at least one vibrating pad, and the at least one LED,
wherein the control unit is configured to provide first actuation signals to the acoustic wave actuator for performing a neovascularization treatment, and configured to provide second actuation signals to the at least one primary vibrating pad and the at least one primary LED for performing a vasodilation treatment;
wherein the penile section of the pad comprises a first recess section and a second recess section together forming a penile shape and configured to respectively cradle a scrotum of the patient and at least a portion of the penile shaft of the patient; and
wherein the second recess section is elongated and concaved extending into the pad.

2. The apparatus of Claim 1, wherein the second recess section configured to cradle at least a portion of the penile shaft of the patient includes at least one of a secondary vibrating pad and a secondary LED.

3. The apparatus of any one of Claims 1 or 2, wherein the perineal section includes at least one of a tertiary vibrating pad and a tertiary LED.

4. The apparatus of any one of the previous Claims, wherein the pouch is made from a flexible material configured to stretch around the penile shaft.

5. The apparatus of any one of the previous Claims, wherein the at least one primary LED includes 40 LEDs, each of the LEDs configured to emit light at a wavelength between 685 nm and 780 nm.

6. The apparatus of any one of the previous Claims, further comprising a memory device storing a treatment profile specifying at least one of: (i) a first time duration, a frequency, an amplitude, and a waveform shape for the acoustic wave actuator; (ii) a second time duration and a light intensity for the at least one primary LED, and (iii) a third time duration, a frequency, an amplitude, and a waveform shape for the at least one primary vibrating pad.

7. The apparatus of Claim 6, wherein the light intensity is specified by at least one of a voltage amplitude and a pulse width modulation percentage.

8. The apparatus of Claim 6 or Claim 7, wherein the treatment profile is received by the control unit wirelessly from a user device or is input by a user via a user interface.

9. The apparatus of any one of the previous Claims, wherein the pouch includes a first side and a second, opposite side, the first side being connected to the pad at the second recess section, and the second side having a greater length compared to the first side.

10. The apparatus of any one of the previous Claims, wherein the pad includes memory foam and a cover.

11. The apparatus of any one of the previous Claims, wherein the control unit transmits the first actuation signals at a first time during a session and the second actuation signals at a second, subsequent time during the session.

12. The apparatus of any one of the previous Claims, wherein the control unit transmits the first actuation signals and the second actuation signals at a same time during a session.

13. The apparatus of any one of the previous Claims, wherein the control unit is configured to provide the neovascularization treatment and the vasodilation treatment for a duration of between 5 minutes and 20 minutes.

## Patentansprüche

1. Einrichtung (100) zur Behandlung erektiler Dysfunktion, umfassend:
ein Polster (102), das geeignet ist, um mit der Gesäßregion eines Patienten in Kontakt zu kommen, wobei das Polster einen Dammabschnitt (202) und einen Penisabschnitt (204) umfasst und so geformt ist, dass es eine Sitz- oder Sattelform bildet, wobei der Dammabschnitt einen Akustikwellenbetätiger (130) einschließt;
einen Beutel (210), der mit dem Penisabschnitt des Polsters verbunden ist, wobei der Beutel konfiguriert ist, um sich um den Penisschaft des Patienten zu wickeln, und wobei der Beutel mindestens ein primäres Vibrationspolster (128) und mindestens eine primäre LED (126) einschließt;
eine Steuereinheit (140), die elektrisch mit dem Akustikwellenbetätiger, dem mindestens einen Vibrationspolster und der mindestens einen LED gekoppelt ist,
wobei die Steuereinheit konfiguriert ist, um erste Betätigungssignale an den Akustikwellenbetätiger zum Durchführen einer Neovaskularisierungsbehandlung bereitzustellen, und konfiguriert ist, um zweite Betätigungssignale an das mindestens eine primäre Vibrationspolster und die mindestens eine primäre LED zum Durchführen einer Vasodilatationsbehandlung bereitzustellen;
wobei der Penisabschnitt des Polsters einen ersten Aussparungsabschnitt (206) und einen zweiten Aussparungsabschnitt (208) umfasst, die zusammen eine Penisform bilden und konfiguriert sind, um jeweils einen Hodensack des Patienten und mindestens einen Abschnitt des Penisschafts des Patienten zu umgeben; und
wobei der zweite Aussparungsabschnitt länglich und konkav ist und sich in das Polster hinein erstreckt.

2. Einrichtung nach Anspruch 1, wobei der zweite Aussparungsabschnitt, der konfiguriert ist, um mindestens einen Teil des Penisschafts des Patienten zu umgeben, mindestens eines von einem sekundären Vibrationspolster (128) und einer sekundären LED (126) einschließt.

3. Einrichtung nach einem der Ansprüche 1 oder 2, wobei der Dammabschnitt mindestens eines von einem tertiären Vibrationspolster und einer tertiären LED (124) einschließt.

4. Einrichtung nach einem der vorstehenden Ansprüche, wobei der Beutel aus einem flexiblen Material besteht, das konfiguriert ist, um sich um den Penisschaft zu dehnen.

5. Einrichtung nach einem der vorstehenden Ansprüche, wobei die mindestens eine primäre LED 40 LEDs einschließt, wobei jede der LEDs konfiguriert ist, um Licht mit einer Wellenlänge zwischen 685 nm und 780 nm zu emittieren.

6. Einrichtung nach einem der vorstehenden Ansprüche, ferner umfassend eine Speichervorrichtung, die ein Behandlungsprofil speichert, das mindestens eines der folgenden Elemente angibt: (i) eine erste Zeitdauer, eine Frequenz, eine Amplitude und eine Wellenform für den Akustikwellenbetätiger; (ii) eine zweite Zeitdauer und eine Lichtintensität für die mindestens eine primäre LED und (iii) eine dritte Zeitdauer, eine Frequenz, eine Amplitude und eine Wellenform für das mindestens eine primäre Vibrationspolster.

7. Einrichtung nach Anspruch 6, wobei die Lichtintensität durch mindestens eines von einer Spannungsamplitude und einem Prozentsatz der Pulsweitenmodulation angegeben wird.

8. Einrichtung nach Anspruch 6 oder Anspruch 7, wobei das Behandlungsprofil von der Steuereinheit drahtlos von einer Benutzervorrichtung empfangen oder von einem Benutzer über eine Benutzerschnittstelle eingegeben wird.

9. Einrichtung nach einem der vorstehenden Ansprüche, wobei der Beutel eine erste Seite und eine zweite, gegenüberliegende Seite einschließt, wobei die erste Seite am zweiten Aussparungsabschnitt mit dem Polster verbunden ist und die zweite Seite im Vergleich zur ersten Seite eine größere Länge aufweist.

10. Einrichtung nach einem der vorstehenden Ansprüche, wobei das Polster eine Memory-Schaum und eine Abdeckung einschließt.

11. Einrichtung nach einem der vorstehenden Ansprüche, wobei die Steuereinheit die ersten Betätigungssignale zu einem ersten Zeitpunkt während einer Sitzung und die zweiten Betätigungssignale zu einem zweiten, nachfolgenden Zeitpunkt während der Sitzung überträgt.

12. Einrichtung nach einem der vorstehenden Ansprüche, wobei die Steuereinheit während einer Sitzung die ersten Betätigungssignale und die zweiten Betätigungssignale gleichzeitig überträgt.

13. Einrichtung nach einem der vorstehenden Ansprüche, wobei die Steuereinheit konfiguriert ist, um die Neovaskularisierungsbehandlung und die Vasodilatationsbehandlung für eine Dauer zwischen 5 Minuten und 20 Minuten bereitzustellen.

## Revendications

1. Appareil de traitement de dysfonction érectile (100) comprenant :
un coussin (102) adapté pour entrer en contact avec une région postérieure d'un patient, le coussin comprenant une section périnéale (202) et une section pénienne (204) est profilé pour former une forme de siège ou une forme de selle, la section périnéale comportant un actionneur d'ondes acoustiques (130) ;
une poche (210) reliée à la section pénienne du coussin, la poche étant conçue pour s'enrouler autour d'une verge du patient, la poche comportant au moins un coussin vibrant primaire (128), et au moins une DEL primaire (126);
une unité de commande (140) couplée électriquement à l'actionneur d'ondes acoustiques, à l'au moins un coussin vibrant, et à l'au moins une DEL,
dans lequel l'unité de commande est configurée pour fournir des premiers signaux d'actionnement à l'actionneur d'ondes acoustiques afin d'effectuer un traitement de néovascularisation, et configurée pour fournir des seconds signaux d'actionnement à l'au moins un coussin vibrant primaire et à l'au moins une DEL primaire afin d'effectuer un traitement de vasodilatation;
dans lequel la section pénienne du coussin comprend une première section évidée (206) et une seconde section évidée (208) formant ensemble une forme de pénis et conçues pour accueillir respectivement un scrotum du patient et au moins une partie de la verge du patient ; et
dans lequel la seconde section évidée est allongée et concave en s'étendant dans le coussin.

2. Appareil selon la revendication 1, dans lequel la seconde section évidée conçue pour accueillir au moins une partie de la verge du patient comporte au moins l'un parmi un coussin vibrant secondaire (128) et une DEL secondaire (126).

3. Appareil selon l'une quelconque des revendications 1 ou 2, dans lequel la section périnéale comporte au moins un coussin vibrant tertiaire et une DEL tertiaire (124).

4. Appareil selon l'une quelconque des revendications précédentes, dans lequel la poche est fabriquée à partir d'un matériau flexible conçu pour s'étirer autour de la verge.

5. Appareil selon l'une quelconque des revendications précédentes, dans lequel l'au moins une DEL primaire comporte 40 DEL, chacune des DEL étant configurée pour émettre de la lumière à une longueur d'onde comprise entre 685 nm et 780 nm.

6. Appareil selon l'une quelconque des revendications précédentes, comprenant en outre un dispositif de mémoire stockant un profil de traitement spécifiant au moins l'une parmi : (i) une première durée, une fréquence, une amplitude, et une forme d'onde pour l'actionneur d'ondes acoustiques ; (ii) une deuxième durée et une intensité lumineuse pour l'au moins une DEL primaire, et (iii) une troisième durée, une fréquence, une amplitude, et une forme d'onde pour l'au moins un coussin vibrant primaire.

7. Appareil selon la revendication 6, dans lequel l'intensité lumineuse est spécifiée par au moins l'un parmi une amplitude de tension et un pourcentage de modulation de largeur d'impulsion.

8. Appareil selon la revendication 6 ou selon la revendication 7, dans lequel le profil de traitement est reçu par l'unité de commande de manière sans fil à partir d'un dispositif utilisateur ou est saisi par un utilisateur par le biais d'une interface utilisateur.

9. Appareil selon l'une quelconque des revendications précédentes, dans lequel la poche comporte un premier côté et un second côté opposé, le premier côté étant relié au coussin au niveau de la seconde section évidée, et le second côté ayant une longueur supérieure par comparaison au premier côté.

10. Appareil selon l'une quelconque des revendications précédentes, dans lequel le coussin comporte une mousse à mémoire de forme et un revêtement.

11. Appareil selon l'une quelconque des revendications précédentes, dans lequel l'unité de commande transmet les premiers signaux d'actionnement à un premier moment pendant une session et les seconds signaux d'actionnement à un second moment ultérieur pendant la session.

12. Appareil selon l'une quelconque des revendications précédentes, dans lequel l'unité de commande transmet les premiers signaux d'actionnement et les seconds signaux d'actionnement à un même moment pendant une session.

13. Appareil selon l'une quelconque des revendications précédentes, dans lequel l'unité de commande est configurée pour fournir le traitement de néovascularisation et le traitement de vasodilatation pendant une durée comprise entre 5 minutes et 20 minutes.
